(19) Europäisches Patentamt
European Patent Office
Office européen des brevets

(11) **EP 2 152 324 B1**

(12) **EUROPÄISCHE PATENTSCHRIFT**

(45) Veröffentlichungstag und Bekanntmachung des Hinweises auf die Patenterteilung:
**14.01.2015 Patentblatt 2015/03**

(21) Anmeldenummer: **08706393.9**

(22) Anmeldetag: **09.03.2008**

(51) Int Cl.:
*A61L 2/10* (2006.01)          *A61L 2/22* (2006.01)
*A01N 59/26* (2006.01)          *A01N 25/00* (2006.01)
*A01M 21/04* (2006.01)

(86) Internationale Anmeldenummer:
**PCT/CH2008/000095**

(87) Internationale Veröffentlichungsnummer:
**WO 2008/101364 (28.08.2008 Gazette 2008/35)**

(54) **PFLANZENSCHUTZVERFAHREN UND -VORRICHTUNG ZUR KONTROLLE UND ELIMINATION VON PFLANZENSCHÄDLINGEN MITTELS ELEKTROLYTISCH HERGESTELLTEN OXIDATIVEN RADIKALEN, UVC-LICHT, UND LUFT UNTERSTÜTZTER ELEKTROSTATISCHER SPRÜHTECHNOLOGIE**

CROP PROTECTION METHOD AND DEVICE FOR CONTROLLING AND ELIMINATING PLANT PESTS USING ELECTROLYTICALLY-PRODUCED OXIDATIVE RADICALS, UVC LIGHT AND AN AIR-ASSISTED ELECTROSTATIC SPRAYING TECHNIQUE

PROCÉDÉ ET DISPOSITIF DE PROTECTION PHYTOSANITAIRE POUR LA LUTTE CONTRE LES PARASITES DES PLANTES À L'AIDE DE RADICAUX OXYDANTS PRODUITS PAR VOIE ÉLECTROLYTIQUE, DE LUMIÈRE UVC ET DE LA TECHNOLOGIE DE PULVÉRISATION ÉLECTROSTATIQUE ASSISTÉE PAR AIR

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.02.2007 CH 2852007**

(43) Veröffentlichungstag der Anmeldung:
**17.02.2010 Patentblatt 2010/07**

(73) Patentinhaber: **Steffen, Hanspeter**
**3421 Rüti b. Lyssach (CH)**

(72) Erfinder: **Steffen, Hanspeter**
**3421 Rüti b. Lyssach (CH)**

(74) Vertreter: **Vinazzer, Edith**
**Schönburgstraße 11/7**
**1040 Wien (AT)**

(56) Entgegenhaltungen:
WO-A-2004/089075          WO-A-2005/082176
CH-B1- 704 641          US-A- 5 040 329

- DATABASE WPI Week 200318 Thomson Scientific, London, GB; AN 2003-178746 XP002490016 -& JP 2002 335764 A (KAWANO H) 26. November 2002 (2002-11-26)

EP 2 152 324 B1

**Beschreibung**

## TECHNISCHES GEBIET

Die Erfindung betrifft ein Pflanzenschutz - Verfahren nach Anspruch 1 und eine Vorrichtung zum Pflanzenschutz nach Anspruch 6.

## STAND DER TECHNIK

Zur Bekämpfung von schädlichen Insekten, Pilzen, Bakterien, Viren und Hefen und anderen Schädlingen wurden bis heute im Pflanzenschutz hochgiftige Chemikalien und Substanzen eingesetzt, die auf Nutzpflanzen giftige Rückstände bilden, Resistenzen bei Schädlingen verursachen und die Umwelt schwer belasten und zudem sehr teuer sind.

Der Einsatz von giftigen Chemikalien im Pflanzenschutz ist daher heute sehr umstritten und Konsumenten bevorzugen preiswerte, biologisch und ökologisch Umwelt freundlich hergestellte pflanzliche Nahrungsmittel ohne giftige Inhaltstoffe oder Rückstände.

Mit dem erfindungsgemässen Verfahren und der erfindungsgemässen Vorrichtung können alle Arten von Pflanzenschädlingen kontrolliert werden, ohne dass giftige und Umwelt schädigende und Resistenzen bildende Substanzen mit Residualwirkung eingesetzt werden müssen.

Die erfindungsgemässe Pflanzenschutztechnik ist sauber, bedeutend billiger, gleichwertig effizient und vor allem Umwelt freundlich und kann auch im ökologischen und Bio-Anbau eingesetzt werden. Zudem erlaubt diese neue Technologie die bessere Miniaturisierung der Spraygeräte, da das Verfahren technisch weniger aufwendig ist als das WO 2004/089075 A2 bekannte aus der Verfahren, wobei ein erstes Spritzgerät zur Vorbenetzung der Pflanzen mit elektrisch negativ aufgeladenem Wasser und ein zweites Spritzgerät zum Aufbringen von ozonhaltigem Wasser mit Hilfe luftassistierter elektrostatischer Spraytechnologie und UVC-Licht-Direktbestrahlung verwendet werden.

## DARSTELLUNG DER ERFINDUNG

Aufgabe der Erfindung ist die Angabe eines neuen kostengünstigen, Umwelt freundlichen und biologischen Verfahrens zum Schutz von Pflanzen gegen schädliche Insekten-Pilze-, Bakterien-, Viren- und Hefen - Befall ohne Rückstände und Resistenzen bildende Chemikalien unter Einsatz von in Wasser, unter Zugabe von Ionen bildenden Salzen, elektrolytisch hergestellten oxidativen Radikalen, UVC-Licht, und mit Hilfe Luft unterstützter elektrostatischer Sprühtechnologie, integriert in einer motorgetriebenen Rücken- oder Handspritze, oder in andere Spritzentypen.

Weiter ist die Aufgabe der Erfindung die Angabe einer Vorrichtung zur Durchführung des Verfahrens.

## EINFÜHRUNG

### Elektrolytisch hergestelltes, oxidatives Wasser (EOW)

Elektrolytisch oxidatives Wasser (EOW) oder chemisch aktives Wasser zerstört Keime, Pilze, und Insekten durch oxidative Radikale nicht chemisch, sondern physikalisch. Wegen seines hohen oxidativen Reduktionspotentials (ORP) beschädigt "AktivesWasser" die Zellwand-Membranen von Pathogenen.
Der Krankheitserreger ist komprimitiert, was zu einer osmotischen oder hydrogenen Überlastung im Zellinneren führt.

Die beschädigten Zellmembranen erlauben einen erhöhten Wassertransfer zwischen den Zellmembranen, was zu einer hydrogenen Überflutung der Zellen führt und diese schneller gefüllt werden, als die Zellen sich des Wassers entledigen können.

Diese Tatsache führt zu einem Zerplatzen der Zellen, respektive zum Zelltod durch Druckexplosion in wenigen Sekunden.

Da es sich um ein physikalisches Zerstörungsprinzip handelt, ergeben sich nachweislich keine Resistenzen bei Pathogenen.

Prinzip der Elektrolyse (verg. Bild Annex 1)

Beispiel einer Elektrolyse mit einer Zinkiodid - Lösung (Elektrodenmaterial beliebig)

Verbindet man zwei Metallplättchen (Elektroden) mit jeweils einem Kabel und einer Vorrichtung, die Gleichstrom erzeugt z.B. einer Batterie oder einem Gleichrichter - und überführt diese Plättchen in ein Becherglas mit wässriger Lösung (beliebige Ionen) und legt nun eine Spannung an, so bildet sich an beiden Metallplättchen ein Stoff, dessen Ionen in der Lösung vorhanden sind.

Die Spannungsquelle bewirkt einen Elektronenmangel in der mit dem Pluspol (Anode) verbundenen Elektrode und einen Elektronenüberschuss in der anderen, mit dem Minuspol (Kathode) verbundenen Elektrode. Die wässrige

Lösung zwischen der Kathode und Anode enthält Elektrolyte, das sind positiv oder negativ geladene Ionen. Die positiv geladenen Kationen in einer Elektrolysezelle wandern durch das Anlegen einer Spannung zur negativ geladenen Kathode (Anziehung entgegen gesetzter Ladungen). An der Kathode nehmen sie ein oder mehrere Elektronen auf und werden dadurch reduziert.

An der Anode läuft der entgegengesetzte Prozess ab. Dort geben die negativ geladenen Anionen Elektronen ab, das heißt sie werden oxidiert. Die Zahl der durch die Reduktion an der Kathode verbrauchten Elektronen entspricht den von der

Anode aufgenommenen Elektronen. Bei der Elektrolyse von wässriger Kochsalzlösung entsteht die gleiche Volumenmenge

Wasserstoffgas wie Chlorgas. Bei der Elektrolyse von Wasser entsteht doppelt so viel Wasserstoffgas wie Sauerstoffgas, da die zwei positiv geladenen Protonen eines Wassermoleküls zur Kathode wandern und dort jeweils ein Elektron aufnehmen müssen, damit sich Wasserstoff bildet, während das doppelt negativ geladene Sauerstoffanion an der Anode gleich zwei Elektronen abgeben muss, um sich zum Sauerstoffmolekül zu verbinden.

Die Spannung, die zur Elektrolyse mindestens angelegt werden muss, bezeichnet man als Abscheidungspotential, bei der Elektrolyse von Wasser oder bei wässrigen Salzlösungen spricht man auch von der Zersetzungsspannung. Diese Spannung (oder eine höhere Spannung) muss angelegt werden, damit die Elektrolyse überhaupt abläuft. Für jeden Stoff, für jede Umwandlung von Ionen zu zwei oder mehratomigen Molekülen kann die Zersetzungsspannung, das Abscheidepotential, anhand des Redoxpotentials ermittelt werden. Aus dem Redoxpotential erhält man noch viele andere wichtige Hinweise für die Elektrolyse,beispielsweise zur elektrolytischen Zersetzung von Metallelektroden in Säure oder zur Verminderung von Zersetzungsspannung durch Abänderung von pH-Werten.

Beispielsweise lässt sich durch das Redoxpotential berechnen, dass die Bildung von Sauerstoff an der Anode bei der Elektrolyse von Wasser in basischer Lösung (Zersetzungsspannung: 0,401 V) unter geringerer Spannung abläuft als in saurer(Zersetzungsspannung:

1,23 V) oder neutraler (Zersetzungsspannung: 0,815 V) Lösung, an der Kathode hingegen bildet sich leichter Wasserstoff unter sauren Bedingungen, als unter neutralen oder basischen Bedingungen).

Sind in einer Elekrolytlösung mehrere reduzierbare Kationen vorhanden, so werden nach der Redoxreihe zunächst die Kationen an der Kathode reduziert, die in der Redoxreihe (Spannungsreihe) ein positiveres (schwächer negatives)Potential haben, die also dem 0 Potential der Proton-Wasserstoff Elektrodenspannung möglichst nahe kommen. Bei der Elektrolyse einer wässrigen Kochsalzlösung bildet sich an der Kathode normalerweise Wasserstoff und nicht Natrium. Auch beim Vorliegen von mehreren Anionenarten, die oxidiert werden können, kommen zunächst diejenigen zum Zuge, die in der Redoxreihe möglichst nahe am Spannungsnullpunkt sind, also ein schwächeres positives Redoxpotential besitzen. Normalerweise entsteht bei der Elektrolyse von wässriger NaCl an der Anode also Sauerstoff und nicht Chlor. Nach Überschreiten der Zersetzungsspannung wächst mit Spannungszunahme proportional auch die Stromstärke. Nach Faraday ist die Gewichtsmenge eines elektrolytisch gebildeten Stoffs proportional zu der geflossenen Strommenge (Stromstärke multipliziert mit der Zeit). Für die Bildung von 1 g Wasserstoff (ca. 11,2 Liter, bei der Bildung eines Wasserstoffmoleküls werden zwei Elektronen benötigt) aus wässriger Lösung wird eine Strommenge von 96485 C (As)=1Faraday benötigt. Bei einer Stromstärke von 1 A zwischen den Elektroden dauert die Bildung von 11,2 Litern Wasserstoff also 26 Stunden und 48 Minuten.

Neben dem Redoxpotential ist noch die Überspannung (das Überpotential) von Bedeutung. Auf Grund von kinetischen Hemmungen an Elektroden benötigt man häufig eine deutlich höhere Spannung als sich dies aus der Berechnung der Redoxpotentiale errechnet. Die Überspannungseffekte können je nach Materialbeschaffenheit der Elektroden auch die Redoxreihe ändern, so dass andere Ionen oxidiert oder reduziert werden als dies nach dem Redoxpotential zu erwarten gewesen wäre. Kurz nach Abschaltung einer Elektrolyse kann man mit einem Amperemeter einen Stromausschlag in die andere Richtung feststellen. In dieser kurzen Phase setzt der umgekehrte Prozess der Elektrolyse, die Bildung einer galvanischen Zelle ein. Hierbei wird nicht Strom für die Umsetzung verbraucht, sondern es wird kurzzeitig Strom erzeugt; dieses Prinzip wird bei Brennstoffzellen genutzt.

Wenn man durch eine Elektrolyse eine Trennung einzelner Moleküle oder Bindungen erzwingt, wirkt gleichzeitig ein galvanisches Element, dessen Spannung der Elektrolyse entgegenwirkt. Diese Spannung wird auch als Polarisationsspannung bezeichnet.

### Elektroden

Es gibt nur wenige Anoden-Elektroden, die während der Elektrolyse inert bleiben - also überhaupt nicht in Lösung gehen. Platin, Kohle resp. <u>Diamant</u> sind Materialien, die sich während einer Elektrolyse überhaupt nicht auflösen. Es gibt auch Metalle, die sich trotz stark negativem Redoxpotentials nicht auflösen. Dies wird als "Passivität" bezeichnet. Eine Eisenanode, die mit konzentrierter Salpetersäure behandelt wurde, löst sich nicht auf und es gehen keine Eisen (II) oder (III)-Kationen in Lösung; sie hat "Passivität".

Hemmungserscheinungen an der Anode, die bei der Sauerstoffbildung zu einer Überspannung führen, beobachtet man bei <u>Diamant</u> und Platinanoden (Überspannung: 0,44 V). Bei diesen entsteht bei der Elektrolyse von wässriger Kochsalzlösung Chlor statt Sauerstoff. An Zink-, Blei-(Überspannung: 0,78 V) und besonders Quecksilberkathoden (0,80 V) zeigen Wasserstoffprotonen eine erhebliche Überspannung und die Bildung von Wasserstoff erfolgt erst bei einer viel höheren Spannung. Die erhebliche Überspannung von Wasserstoff an der Quecksilberkathode, in der das Natrium als Amalgam gebunden wird und daher dem Gleichgewicht entzogen wird, nutzt man zur technischen Herstellung von Natronlauge. Durch die erhebliche Überspannung an dieser Elektrode bei der Wasserstoffbildung ändert sich die Redoxreihe und statt Wasserstoffprotonen wandern nun Natriumkationen zur Quecksilberkathode.

Elektrolyse von Wasser

Die Elektrolyse von Wasser besteht aus zwei Teilreaktionen, die an den beiden Elektroden ablaufen. Die Elektroden tauchen in Wasser ein, welches durch die Zugabe von etwas Kochsalz besser leitend gemacht wird, wobei dann anstatt Sauerstoff Chlor gewonnen wird.

Positiv geladene Hydronium-Ionen ($H_3O^+$) wandern im elektrischen Feld zu der negativ geladenen Elektrode (Kathode), wo sie jeweils ein Elektron aufnehmen. Dabei entstehen Wasserstoff-Atome, die sich mit einem weiteren, durch Reduktion entstandenen H-Atom zu einem Wasserstoff-Molekül vereinigen. Übrig bleiben Wasser-Moleküle.

$$2 H_3O^+ + 2 e^- \rightarrow H_2 + 2 H_2O$$

Der abgeschiedene, gasförmige steigt an der Kathode auf.

Zur positiv geladenen Elektrode (Anode) wandern die negativgeladenen Hydroxid-Ionen.

Jedes Hydroxid-Ion gibt ein Elektron an den Plus-Pol ab, so dass Sauerstoff-Atome entstehen, die sich zu Sauerstoff-Molekülen vereinigen resp. bei NaCl Zugabe zu Chlor-Molekülen.

Die übrig bleibenden $H^+$-Ionen werden umgehend von Hydroxid-Ionen zu Wasser-Molekülen neutralisiert.

$$4OH^- \rightarrow O_2 + 2H_2O + 4e^-$$

Auch hier steigt der abgeschiedene Sauerstoff als farbloses Gas an der Anode auf. Die Gesamtreaktionsgleichung der Elektrolyse von Wasser lautet:

$$4 H_3O+ + 4 OH^- \rightarrow 2 H_2 + O_2 + 6 H_2O$$

Die auf der linken Seite stehenden Hydronium- und Hydroxid-Ionen entstammen der Autoprotolyse des Wassers:

$$8 H_2O \rightarrow 4 H_3O^+ + 4 OH^-$$

Man kann die Elektrolysegleichung daher auch folgendermaßen schreiben:

$$8H_2O \rightarrow 2H_2 + O_2 + 6H_2O$$

bzw. nach Kürzen des Wassers:

$$2 H_2O \rightarrow 2 H_2 + O_2$$

Hydroxidion

Das Hydroxidion ist ein negativ geladenes Ion, das entsteht, wenn Basen mit Wasser reagieren. Seine chemische Formel lautet $OH^-$.

Eine allgemeine Base B reagiert nach folgendem Schema mit Wasser: $B + H_2O \rightleftharpoons HB^+ + OH^-$ Anhand der Konzentration der Hydroxidionen kann man den pH-Wert der entstandenen Lösung ermitteln. Dazu berechnet man erst den so genannten pOH-Wert.

$pOH = -\log c(OH^-)$

Und daraus den pH-Wert: $pH = k - pOH$ Zu jeder Temperatur gibt es jeweils ein k. Unter Normbedingungen ist k= -14.

Hydroxidionen sind auch in reinem Wasser bei 20°C in einer Konzentration von $10^{-7}$ mol $l^{-1}$ enthalten. Das hängt mit der Autoprotolyse des Wassers nach folgender Reaktionsgleichung zusammen: $H_2O + H_2O \rightleftharpoons H_3O^+ + OH^-$ Zulassung Die innovative Verwendung von Diamantelektroden - Technologie in der Elektrolyse erhielt vor kurzem grosse Aufmerksamkeit von zahlreichen Universitäts- - Forschungsteams für den Einsatz in der Oberflächen - Desinfektion von Geflügel, Fisch und Fleisch, von Früchten und Gemüse in der Verarbeitungsindustrie . Frühe eigene Versuche und Versuchsergebnisse führten zur Einreichung von Bewilligungsgesuchen bei der FDA (Food and Drug Administration, USA), welche im Dezember 2002 die Bewilligung für die neue Technologie erteilte und mit dem Status"GRAS" (Generally Regarded as Safe) auszeichnete.

Elektrolysiertes oxidatives Wasser erhielt FDA (USA Food and Drug Administration, USDA (United Status Department of Agriculture) und EPA (USA Environmental Protection Agency) - Zulassung für allgemeine Applikationen im Nahrungsmittel-Bereich, für die Nahrungsmittel-Oberflächen Desinfektion, für Milch-, Fleisch- und Restaurant- technische Anwendungen.

Die entsprechenden Seiten der Bewilligungsnummern der FDA und USDA lauten 21 CFR 173,178,182,184 & 198.

Die EPA Bewilligungs- und Publikations-Seite lautet 40 CFR 180.940 und die des National Organic Programms ist 21 CFR 178.1010.

UVC LICHT

UVC Licht, als elektromagnetische Direktstrahlung, hat im Wellenbereich von 254 Nanometer optimale biozide Wirkung gegen Pilze, Bakterien, Hefen, Viren und Insekten und transformiert, in feuchter Umgebung, oxidative Radikale wie Ozon, H2O2 etc. zu hochreaktiven Hydroxy-Radikalen um, die zu einer Oxidation von organischer Substanz führen.

Die UVC Direktstrahlung bewirkt bei Mikroorganismen die Auflösung von Zellmembranen und zerstört DNS-Strukturen.

Im Wellenbereich von 185 Nanometer erzeugt UVC Strahlung Ozon, welches unter hoher Luftfeuchtigkeit ebenfalls hoch oxidative labile Hydroxilverbindungen erzeugt, die mittels der entstehenden Hydroxy-Radikalen die Wirkung von oxidativen Radikalen wie $O_3$, $H_2O_2$ als Biozide wesentlich verstärken (mikrobielles Hürde-Prinzip).

Zulassung

UVC Bestrahlung von Lebensmitteln ist seit 1997 von der FDA und 1998 vom USDA in den USA und in vielen anderen Ländern weltweit zugelassen und für pflanzliche Lebensmittel ebenfalls in der Deutschen Strahlenschutzverordnung.

Für die Effizienz der UVC Strahlung ist die Strahlendosis entscheidend, die in mW/sec/cm 2 (Milli Watt pro Sekunde pro Quadrat- Zentimeter bestrahlter Fläche) angegeben wird.

Die Dosis zur Eliminierung von Mikroorganismen beträgt vorzugsweise 4'000 bis 15'000 mW/sec/cm 2 (abhängig von Art), für Schadinsekten ca. 500'000 -1'500'000 mW/sec (abhängig von Art).

Der Erfinder hat in 5-jähriger Forschungsarbeit im Labor und in Feldversuchen die Effizienz des neuen Pflanzenschutz - Verfahrens mittels in Wasser, unter Zugabe von Ionen bildenden Salzen, elektrolytisch hergestellten oxidativen Radikalen, UVC Licht und mit Hilfe Luft unterstützter elektrostatischer Sprühtechnologie, integriert in einer motorgetriebenen Rücken- oder Handspritze oder anderen Spritzentypen, zusammen mit der entsprechenden Applikations- - Technologie bestätigt.

Das neue Pflanzenschutz - Verfahren erreichte in statistisch relevanten Feldversuchen 94% Effizienz in Mischinfektionen und gleichzeitigem Befall von drei Insektenarten in Weizen und Buschbohnen, Tomaten und Gurken im Freiland - Anbau und im Gewächshaus. Andere Kulturen, in denen die Erfindung getestet wurde und die präventiv behandelt wurden, zeigten keine Ertrags mindernde Schäden.

Nach Kenntnisstand des Erfinders sind bis heute keine wissenschaftlichen Arbeiten auf dem Gebiet des Pflanzenschutzes mittels in Wasser, unter Zugabe von Ionen bildenden Salzen, elektrolytisch hergestellten oxidativen Radikalen, kombiniert mit UVC Licht, und mit Hilfe Luft unterstützter, elektrostatischer Sprühtechnologie, integriert in einer motorgetriebenen Rücken- oder Handspritze oder anderen Spritzentypen, zusammen mit den entsprechenden Applikations- Technologien für den praktischen Feldeinsatz veröffentlicht.

**DIE LÖSUNG DER AUFGABE**

Die Lösung der Aufgabe ist durch die Merkmale der unabhängigen Patentansprüche 1 und 6 definiert.

Das erfindungsgemässe Spritzverfahren und die erfindungsgemässe Vorrichtung zeigen zur Anwendung im Pflanzenschutz gegen schädliche Pilze, Hefen, Bakterien, Viren, Sporen, Protozooen und Schadinsekten die Art und Weise der Biozide, insbesondere der spezifischen Eigenschaften des elektrolysierten, oxidativen Wassers, dessen Herstellung dessen Salzkonzentration und Salzzusammensetzung, dessen Redoxpotential, respektive dessen Konzentration in freien oxidativen Radikalen und Gesamtkonzentration der oxidativen Radikale, und dessen pH-Wert und Aufwandmenge für einen effizienten Spritzvorgang auf.

Gemäss der Erfindung zeigt das Verfahren zudem die Funktionsweise der Biozid-Wirkung und Katalysator- und Insektenfallen-Funktion der UVC Strahlung und dessen Herstellung auf und die Art und Weise der elektrostatischen, luftassistierten Applikations-Technologie für einen effizienten Einsatz auf. Die Vorrichtung kann in ein integriertes System eingebaut werden in welchem die technischen Komponenten zur oxidativen Radikal - Herstellung im Wasser, die UVC-Erzeugung und die elektrostatische, luftassistierte Spraytechnologie mit der entsprechenden Applikations-Technologie in Form einer motorisierten Rückenspritze, einer Handspritze oder anderer Spritzentypen integriert sind.

Dabei liegt der Schwerpunkt der Innovation nicht nur in der Kombination von elektrolytisch oxidativem Wasser (aktivem Wasser) und UVC-Strahlung als neue Anwendung und Hürdentechnologie zur Schädlingsbekämpfung im Pflanzenschutz, sondern auch in der kombinierten neuen Applikationstechnik der luftassistierten elektrostatischen Spraytechnologie und vor allem in der Zusammensetzung der geeigneten Salzverbindungen, die für die Bio-Produktion zugelassen sind, die keine phytotoxischen Schäden an Pflanzen verursachen und zudem in elektrolytischer Form eine optimale Wirkung als Biozide gegen Insekten und andere Pflanzenschädlinge haben.

In 5-jährigen Labor- und Feldversuchen wurden diese geeigneten Salzkombinationen und oxidativen Radikal - Konzentrationen im Sprühwasser vom Erfinder empirisch und praktisch eruiert, getestet und optimiert für über 30 verschiedene landwirtschaftliche und gärtnerische Pflanzenarten. Die neue kombinierte Applikationstechnik, zusammen mit den korrekten Salzkombinationen und den entsprechenden Konzentrationen der oxidativen Radikale sind essentiell für den erfolgreichen Einsatz von elektrolytischen oxidativen Radikalen im Wasser und der UVC-Bestrahlung im Pflanzenschutz und erfüllen alle Parameter zur optimalen Wirkungsweise des neuen Pflanzenschutz - Verfahrens. Ohne die spezielle Anwendung der elektrostatischen Sprühtechnologie, die eine vollständige Benetzung aller Pflanzenteile erlaubt und damit die Desinfektion durch die "Kaltoxidation" von Pathogenen und die Eliminierung von Insekten garantiert, ist die kombinierte Wirkung von elektrolytisch oxidierendem Wasser und UVC-Strahlung ungenügend, da die Zerstörung der Pathogene bei einer partiellen Desinfektion nicht garantiert ist.

Weiter ist die Erfindung innovativ bezüglich der Herstellung der oxdativen Radikale mittels zweier Verfahren, der Elektrolyse in der Diazelle mit Diamant beschichteten Elektroden und der Zylinder-Elektrolyse mit Platin elektroden, die bei der Elektrolyse von salzhaltigem Wasser mit $Na^+$ und Cl-Ionen, vor allem Cl- Ionen produzieren und nicht $H^+$Ionen. Dieser Umstand ermöglicht die Produktion von organisch abbaubaren Hypo-Chlorid- Verbindungen (HOCL) oder Hypo-Chlorid Säure $H_2CLO$. Zudem produziert die Zylinder-Elektrolyse, dank ihrem speziellen Aufbau und den Platinelektroden, mehr aktiven Sauerstoff (03).

Die Erfindung des neuen Pflanzenschutz - Verfahrens besteht essentiell in der Kombination der elektrolytischen Herstellung von oxidativen Radikalen in Wasser in zwei verschiedenen Herstellungsverfahren (Biozid), deren Mischung und Zwischenlagerung im Tank und der anschliessenden Ausbringung der Biozide mittels einer Pumpe und mit Hilfe der Luft assistierten elektrostatischen Spraytechnologie und direktem UVC-Licht als Biozid und ReaktionsKatalysator, der als Beschleuniger der Oxidationsprozesse dient und zu einer Oxidation der Pathogenen führt. Ein Ausführungsbeispiel der Vorrichtung zum erfindungsgemässen Pflanzenschutz mit elektrolytisch oxidativen Radikalen und UVC-Bestrahlung unter Mithilfe von elektrostatischer Spraytechnologie besteht aus folgenden technische Komponenten:

1. Motorisierte Rückenspritze oder Handspritze oder anderer Spritzentyp.

2. Eine oder mehrere elektrostatische Spraydüsen mit Niederdruck und grossvolumiger Luftunterstützung zur Feintropfen-Erzeugung und eingebauter Elektrode zur induktiven Aufladung der Spraytropfen mit einer Tropfengrösse von idealer weise nicht mehr als 50 Mikron, die eine Aufladung der Mikrotropfen von ca. 600 V erlaubt, inklusive der entsprechenden Elektronik zur Erzeugung des DC - Ladungsstroms der Elektroden und automatischer oder Handsteuerung zur Aktivierung oder Deaktivierung der Elektroden.

3. Spraylanze oder Spraybalken oder Spraygebläse enthaltend eine oder mehrere elektrostatische Spraydüsen in variabler, den Bedürfnissen entsprechender Anordnung.

4. Ein oder mehrere Speicher - Wassertanks für die Aufnahme der elektrolytischen oxidativen Radikale im Wasser in den Volumen - Dimensionen der entsprechenden Spritzentypen, idealer weise von 1 - 4000 Litern oder mehr.

5. Eine oder mehrere Umwälzpumpen, gemäss der zu erbringenden Literleistung pro Stunde mit einer Minimum Druckleistung von 4 Atm. inklusive elektronische Steuerung mit " on" und " off" Schalter, inklusive oxidatonsfreien Leitungen aus Viton, Teflon oder PVC oder einem entsprechend anderen geeigneten Material.

6. Eine oder mehrere Sprühpumpen, zur Ausbringung der erforderlichen Sprühmenge von 50 - 600 Liter pro Hektar oder mehr mit " on" und "off" Schalter, inklusive oxidatonsfreien Leitungen aus Viton, Teflon oder PVC oder einem entsprechend anderen geeigneten Material.

7. Zwei oder mehr Druckmanometer und Druckkontrollventile mit Rücklauffunktion, eine für die Umwälzpumpe und eine für die Spraypumpe.

8. Ein Luftdruckmesser

9. Zwei Redox - Messgeräte für die Messung der oxidativen Radikalen - Konzentration im Tank und in den Zubringerleitungen zu den elektrostatischen Spraydüsen.

10. Eine oder mehrere Diacell-Elektrolysezellen mit je ein bis drei oder mehr Elektrolysekammern, je nach Bedarf, mit Volumenflussmesser und Flusssonde und entsprechendem Steuergerät mit manueller und automatischer Kathoden- und Anoden - Lastumkehr, eingebautem Amperemeter und Voltmeter und Lampenfunktionskontrolle, mit automatischer Abschaltung ohne Volumenfluss, inklusive Leitungen und Anschlüssen und Kontrollhahn und Probeentnahmestelle.. (220 oder 340 V)

11. Eine oder mehrere Zylinder-Elektrolysezellen mit Plattenelektroden und Diaphragmazellen - Trennung mit Anode und Kathode mit Umkehrfunktion zur Produktion von saurem und basischem Elektrolysewasser mit anionischen und kationischen oxidativen Radikalen, mit elektrischer Steuerung, Strompulsor und Sicherung mittels Steuergerät mit manueller und automatischer Kathoden- und Anoden - Lastumkehr, eingebautem Amperemeter und Voltmeter und Lampenfunktionskontrolle, mit automatischer Abschaltung ohne Volumenfluss, inklusive Leitungen und Anschlüssen und Kontrollhahn und Probeentnahmestelleg (220 oder 340 V), inklusive Redox-Messgerät für die anodische und katodische Elektrolytenflüssigkeit, inklusive elektronischer Mischbatterie, die zur Einstellung des gewünschten pH- Wertes des elektrolytischen oxidativen Wassers (EOW) dient.

12. Eine oder mehrere UVC Lampen von 30 - 330 Watt oder mehr als Rund- Oval - oder Stablampen konzipiert , in wasserdichtem Quarzmantel und wasserdicht verschraubt mit elektrischen Vorschaltgeräten in Sicherheits- oder Steuergehäuse integriert mit Kontrolllampen und "on" und "off" Schaltern und Alarm-Funktionssteuerung mit Sicherungsschalter inklusive reflektierendem Strahlungsschutz.

13. Luftgenerator als Gebläse oder Kompressor konzipiert, mit Leitungen und Zapfwellen- oder elektrischem oder motorisiertem Antrieb je nach Leistungsbedarf, inklusive Druck- und Volumenmeter und entsprechenden Leitungen und Kühlaggregaten mit Ventilator und Wasserkühlung mittels Kühlradiator.

14. Stromquelle aus Steckdose oder Batterie, aus der Sonnen-Energieversorgungsanlage oder aus Stromgenerator, individuell oder per Zapfwellenantrieb erzeugt inklusive Steuerungen und Sicherungen.

Das innovative Anwendungsverfahren der Erfindung beinhaltet drei wesentliche Schritte:

1. Herstellung der bioziden oxidativen Radikale in wässeriger, salzhaltige Lösung mittels Elektrolyse.

2. Spritzen der hergestellten bioziden wässrigen Lösung von oxidativen Radikalen auf die zu behandelnden Pflanzen mit Hilfe der elektrostatischen Luft assistierten Düsentechnik.

3. Direktbestrahlung der Pflanzen mit biozidem UVC-Licht und gleichzeitiger Injizierung der Katalysatorwirkung auf die oxidativen Radikale zur Oxidations- Prozessbeschleunigung und als Stressor zur Auslösung einer systemisch erlangten Resistenz in Pflanzen.

1. Herstellung der bioziden oxidativen Radikale in wässeriger, salzhaltiger Lösung mittels Elektrolyse.

Die Herstellung der bioziden oxidativen Radikalen in wässriger, salzhaltiger Lösung geschieht durch zwei verschiedene, aber sich ergänzenden Elektrolyseverfahren.

Das erste Verfahren wird implementiert mit der Diacell-Elektrolyse mittels Diamant- beschichteten Elektroden. Dabei entsteht ein Cocktail aus oxidativen Radikalen nahe am "neutralen Bereich" mit einem pH - Wert von 6,4 bis 6,8. An der Anode werden neben OH- Hydroxylgruppen und 03 vor allem freies Chlor (Cl-) gebildet, die mit den Hydroxylgruppen zur Bildung von Hypochlorid HOCL und Hypochlorid Säure H2OCL führen, die organisch sehr rasch abgebaut werden. Um die Elektrolyse des Wassers bezüglich Stromverbrauchs günstiger und besser durchführen zu können, werden dem Wasser wegen der verbesserten Elektrokonduktivität verschiedene Salze zugemischt, die im Bio-Anbau bewilligt sind.

Bei der Elektrolyse dieser Salzverbindungen entstehen zudem oxidierende reduzierendes Peroxyd-Di-Sulfat, Peroxyd- DiPhosphat und Percarbonat.

Diese Salze sind zum Beispiel pro Liter Spritzflüssigkeit: (für Jungpflanzen)

1.5 gr. NaCl (Kochssalz)

0.3 gr. K2S04 (Kaliumsulfat)

0.3 gr. Na3P04 (Natriumphosphat)

0.1 gr. Mg2SO4 (Magnesiumphosphat)

Diese Salzlösung muss nach der erfolgten Elektrolyse eine Konzentration von min. 35 ppm oder 35mg pro Liter oxidative Radikale als Gesamttotal aufweisen oder ca. 17 ppm oder 17 mg pro Liter freie Radikale enthalten.

Diese Salze sind zum Beispiel pro Liter Spritzflüssigkeit: (für im Wachstum befindliche und ausgewachsene Pflanzen):

2.25 gr. NaCl (Kochssalz)

0.45 gr. K2S04 (Kaliumsulfat)

0.45 gr. Na3P04 (Natriumphosphat)

0.2 gr. Mg2S04 (Magnesiumphosphat)

Diese Konzentrationen können je nach Pflanzenart und Wachstumsstadium und Infektionsdruck verschieden sein!

Diese Salzlösung muss nach der erfolgten Elektrolyse eine Konzentration von min. 90 ppm oder 90mg pro Liter Oxidative Radikale als Gesamttotal aufweisen oder ca. 45 ppm oder 45 mg pro Liter freie Radikale enthalten.

Diese Salzzusammensetzungen können je nach Pflanzenart und Einsatzzweck auch verschieden sein, sowohl als Salze als auch in deren Konzentration.

Das zweite Verfahren wird implementiert mit der Zylinder-Elektrolyse mit Diaphragma, wo die Elektrolysezellen voneinander getrennt sind, bestehend aus einer Anoden-kammer und einer Kathodenkammer. An der positiven Anode aus Platin bilden sich Säure bildende negativ aufgeladene Anionen in einem sauren Bereich von ca. 2.4 pH mit negativer Ladung, und an der negativen Kathode bilden sich Basen bildende positive Kationen in einem alkalischen Bereich von ca. 11 pH mit einer positiven Ladung.

Diese zwei sauren und alkalischen wässrigen Elektrolyselösungen können jetzt beliebig gemischt werden und je nach Einsatz und Infektionsdruck oder Insektenbefall auf die Pflanzen gesprüht werden.

Bei der Elektrolyse von reinem Wasser ohne Salz, werden folgende oxidativen Radikale gebildet:

ELECTROLYTISCHER PROCESS von Wasser

Es entstehen die verschiedensten oxidativen Radikale, wenn Wasser (H2O) elektrolysiert wird zum Beispiel: (E0 ist das Standart Redox-Potential)*:

$$O_2 + H + e^- \; HO_2 \quad E_0 = -0.13 \text{ V} \qquad [1]$$

$$2H^+ + 2e^- \; H_2 \quad E_0 = 0.00 \text{ V} \qquad [2]$$

$$HO_2 + H^+ + e^- \; H_2O_2 \quad E_0 = +1.50 \text{ V} \qquad [3]$$

$$O_3 + 2H^+ + 2e^- \; O_2 + H_2O \quad E_0 = +2.07 \text{ V} \qquad [4]$$

$$OH^- + H^+ + e^- \; H_2O \quad E_0 = +2.85 \text{ V} \qquad [5]$$

$$H_2O + e^- \; H^+ \; OH^- \quad E_0 = -2.93 \text{ V} \qquad [6]$$

$$OH^+ e^- \; OH^- \quad E_0 = +2.02 \text{ V} \qquad [7]$$

ELECTROLYTISCHER PROCESS von Wasser mit Salz NaCL

An der Kathoden - Seite

$$Na+ + e- \; Na$$

$$2Na + 2H2O \; 2Na+ + 2OH- + H2$$

An der Anoden - Seite

$$2Cl- - 2e- \; Cl2$$

Es muss hier erwähnt werden, dass Cl2 (Chlorgas) and OHwie folgt reagieren:

$$Cl \; 2 + 2OH- \; Cl \; O- + Cl- + H2O$$

oder

$$Cl \; 2 + OH- \; HClO + Cl-$$

2. Spritzen der hergestellten bioziden wässrigen Lösung von oxidativen Radikalen auf die zu behandelnden Pflanzen mit Hilfe der elektrostatischen luftassistierten Düsentechnik.

Um die Pflanzen vollständig mit oxidativen Radikalen in der Desinfektionsfüssigkeit zu benetzen, ist eine spezielle Spritztechnologie notwendig.

Die elektrostatische Spritztechnologie garantiert ein vollständiges Benetzen der Pflanzen auf Blattober und unterseite, am Stel und auch an schwer zugänglichen Stellen.

Das Wassertröpfchen, das durch einen ultraschnellen Luftstrom bis nahe an die Schallgeschwindigkeit produziert wird und das nicht grösser als 50 Mikron ist, wird durch eine in Keramik eingebettete Stahlelektrode mit ca.1200 Volt induktiv aufgeladen.

Das Tröpfchen erhält somit eine Ladung von ca. 600 V und 3-5 mOhm pro Liter (Milli Ohm), was einer Anziehungskraft von ca. 75 G entspricht (75-mal sein Eigengewicht).

Das Wassertröpfchen wird somit von allen Teilen der Pflanze angezogen und es entsteht ein feiner Film auf der ganzen Pflanzenoberfläche, der dank der oxidativen Radikale in der Sprayflüssigkeit zu einer Desinfektion der Pflanze führt, respektive zur Zerstörung von Mikroben und Pathogenen und auch einen oxidierenden Effekt auf Insekten ausübt

Verg. Annex 1 und 2

3. Direktbestrahlung der Pflanzen mit biozidem UVC-Licht und gleichzeitiger Injizierung der Katalysator - Wirkung auf die oxidativen Radikale zur Oxidations- - Prozessbeschleunigung und als Stressorauslösender Faktor für eine Gen elizitierte sytemische Resistenz in Pflanzen

Die UVC-Direktbestrahlung der Pflanzen hat vier verschiedene Hauptfunktionen:

1. Direktbestrahlung durch UVC-Licht als Biozid zerstört Zellkern- und Nukleotiden- Funktionen durch Basenverklebung auf der DNS-Helix, was die Zellteilung und methabolischen Prozesse in pathogenen Zellen zum Stillstand bringt und diese deshalb eingehen. Auf die Pflanzenzellen hat das bei kurzer Exposition der Pflanzen zum UVC-Licht keine schädigenden Wirkungen, da Pflanzenzelle vielschichtig und stärker gebaut sind.

2. Die UVC-Bestrahlung hat auch den Zweck, als Katalysator gewisse Zersetzungsmechanismen bei der Bildung von oxidativen Radikalen, im Speziellen bei der Bildung von reaktiven und oxidativen Hydroxyl-Gruppen reaktionsschnell mitzuhelfen.

So zum Beispiel zerstört UVC-Licht im Wellenbereich von 254 nm (Nanometer) Ozon O 3, das in wässeriger Lösung das erste Zwischenprodukt, nämlich H2O2 bildet (Peyton und Glaze 1988).

$$O3 + H2O \; 6 \; O2 + H2O2$$

H2O2 (Wasserstoffperoxid) ist eine schwache Säure. Wenn mit Wasser zusammengebracht dissoziiert H2O2 in Hydroperoxid Ionen:

$$H2O2 + H2O \, \Xi \, HO2\text{-} + H3O+$$

ka = 10-11.6

Unter dem Einfluss von UVC-Licht, zerfallen Ozon und H2O2 , das erste schneller, das zweite etwas langsamer.

Bei diesem Zerfall entstehen eine Vielzahl von Sauerstoff enthaltenden, hochreaktiven oxidativen Radikalen, das häufigste auftretende ist ein hydroxil-freies Radikal (HO=) (Hoigné, 1988).

All diese freien oxidativen Radikale haben sehr kurze Halbwertszeiten (Nanosekunden) und oxidieren organische Substanz sehr rasch.

Das Oxidationpotential von molekularem Ozon, 03, ist 2.07 eV, während das vom hydroxyl-freien Radikal, HO=, 2.83 eV ist.

3. Die dritte Funktion des UVC-Lichts ist die Gen elizitlerte Auslösung einer SAR (Systemic Acquired Resistance), einer systemischen Immunschutzreaktion der Pflanze.

Das intensive und Energie reiche UVC-Licht bewirkt in der Pflanze die gleichen Abwehrreaktionen wie bei einer Attacke eines Insektes oder eines Pilzes.

In komplizierten chemischen Kaskaden - Reaktionen bildet die Pflanze auf drei bekannten chemischen Fährten (Salicil-und Jasmonic - Säure- und Ethylen - Pfade) verschiedenste Abwehrstoffe und Abwehrmechanismen gegen Pathogene, wie Phytoalexine, Phenole Therpentene, Kumarine, Isoflavonoide, Grapevine Reservatrol etc.

Diese Stoffe führen einerseits zur Zerstörung der Pathogenen oder wirken als Repellent für Insekten etc.

Die Pflanzen bildet also innerlich systemische Stoffe zu ihrem Schutz. Dabei verstärkt sich bei jeder Behandlung mit UVC-Licht der immunisierende Schutz der Pflanze von innen. Diese Abwehrmechanismen sind in den meisten Pflanzen genetisch verankert und sind meistens phenotypisch indifferent in der Schädlingsbekämpfung, das heisst, alle "Verteidigungssysteme" gegen Eindringlinge und Feinde werden gleichzeitig mobilisiert.

Diese Verteidigunasmechanismen benötigen von der Pflanze sehr viel Eigenenergie. Eine gute Versorgung mit Nährstoffen und Wasser, ohne weitere Stressfaktoren, sind deshalb Bedingungen für die erfolgreiche Anwendung der UVC-Immunschutztherapie.

4. Die 4. Funktion der UVC-Bestrahlung wirkt sich bei Spritzungen in der Nacht aus.

Wenn die UVC-Lampen angeschaltet sind, wirken sie wie ein Magnet für fliegende Nacht aktive Insekten (keine Bienen und Wespen als Nützlinge).

Fliegende Insekten werden vom intensiven Licht angezogen und durch die starke UV-Strahlung so geschädigt, dass diese eingehen. Damit werden Elterntiere von Faltern z.B. abgefangen und vernichtet, bevor sie ihre Eiablage tätigen können und fressende Raupen im Pflanzenbestand Schäden anrichten können.

### AUSFÜHRUNG DER ERFINDUNG

Zur Ausführung des erfindungsgemässen Pflanzenschutz - Verfahrens zur Kontrolle von Pilz-Bakterien-, Viren- und Insektenbefall in landwirtschaftlichen und gärtnerischen Kulturen, mittels elektrolytisch hergestellten oxidativen Radikalen, UVC-Bestrahlung und mit Hilfe luftassistierter elektrostatischer Spraytechnologie soll die Erfindung am Beispiel einer motorisierten Rückenspritze erläutert werden.

Die gleiche Technik kann auch leistungsangepasst in andere Spritzentypen integriert werden.

Als Grundspritzentyp wird in dieser speziellen Ausführung des Verfahrens eine motorisierte Rückenspritze, wie sie von der Firma STIHL oder von HONDA auf dem Markt angeboten wird, folgendermassen auf die Phyt03 Pflanzenschutz - Technologie angepasst und umgebaut. (verg. Zeichnung A)

Hier die technische Bebeschreibung der STIHL Rückenspritze Modell 420 und ihre Leistungsdaten:

| Technische Daten | |
| --- | --- |
| Hubraum | 56,5 cm$^3$ |
| Gewicht mit Sprührohr | 11,1 kg |
| Max. Luftleistung ohne Blasanlage | 1260 m$^3$/h |

(fortgesetzt)

| Technische Daten | |
|---|---|
| Reichweite horizontal | 12 m |
| Sprühmittelbehälterinhalt | 131 |
| Tankinhalt | 1,51 |
| Reichweite vertikal | 11,5 m |

Die Rückenspritze wird zusätzlich, in einer möglichen ersten Version, mit einem kleinen Dynamo zur Stromerzeugung oder in einer zweiten möglichen Version mit einer 180 W Cadmium 12 V Batterie mit Volt-Transformer ausgerüstet.

Diese Stromversorgung ermöglicht den Anschluss einer UVC-Lampe von min. 30 W Strahlungsleistung.

Die UVC-Lampe, die vorzugsweise als Oval-Bogen-Stab- oder Kreislampe konzipiert ist, wird mittels einem Leichtgewicht - Gestänge mit Halterung aus PVC, Karbonfiber oder aus Leichtmetall oder anderen Materialien mit einem Abstand von vorzugsweise 80 cm oder weniger oder mehr mit dem Sprührohr der Rückenspritze verbunden. Die UVC-Lampe wird mit einem Stromkabel mit der Stromquelle verbunden. Am Kabel ist in günstiger ergonomischer Position ein Schalter angebracht, um die Lampe ein- oder auszu schalten.

Die UVC-Lampe verfügt über einen vorzugsweise kleinen Reflektor, vorzugsweise aus poliertem, rostfreiem Feinblech oder anderen geeigneten Materialien, der gleichzeitig als Schutzschild für den Operateur gegen die UVC-Strahlung dient. (verg. Zeichnung D)

An der Spritze wird, in einer ersten möglichen Version der Ausführung, eine Minlatur-Elektrolysezelle montiert und in das Flüssigkeitsrücklaufsystem integriert und mit der dazugehörenden elektrischen Steuerung und mit der Stromversorgung verbunden. In die Flüssigkeitsrücldauf-Leitung wird ebenfalls ein Flussmeter eingebaut mit elektronischer Sensorsteuerung am Sichtglas, das die Elektrolysezelle bei Wasserfluss einschaltet und ohne Wasserfluss ausschaltet. Diese Zelle ist mit einer genügenden Leistung konzipiert, dass die 13 Liter Spritzflüssigkeit in ca. 15 Minuten elektrolytisch aufbereitet werden kann. Diese Aufgabenlösung der Technik ist für Hobbygärtner und Gartenbesitzer geeignet!

In einer zweiten möglichen Version der Ausführung, für den professionellen Einsatz einer auf die neue Technologie umgerüsteten Rückenspritze, empfiehlt sich die Sprayflüssigkeit in einem separaten Elektrolysegerät herzustellen, das je nach Bedarf 50 - 100 Liter der elektrolysierten oxidativen Lösung herstellt. Für diese Anwendungslösung sind ein Auffangtank von beliebiger Grösse und zwei Ventilhähne für den Zufluss und Abfluss und eine Umwälzpumpe mit vorzugsweise 100 Liter Stundenleistung und mit einem Druck von vorzugsweise 4 bar, inklusive elektrische Steuerung und Stromkabel-Anschluss 220V/50 Hz nötig und eine Elektrolysezelle mit Diamantelektroden-Beschichtung inklusive elektronischer Steuerung und/oder eine Zylinder-Elektrolyse-Apparatur mit Platinelektroden inklusive Steuerung notwendig, oder auch beide gleichzeitig je nach Bedarf und Einsatz und Produktionsleistung. (verg. Zeichnungen B und C)

An der Spitze des Sprührohres wird die elektrostatische Düse mit entsprechender rostfreier Halterung angebracht, die mit einem Luftschlauch durch das Innere des Sprührohres mit dem Luftgebläse der Rückenspritze verbunden wird. Ein oder zwei variable Druckregulierventil - Hähne regeln die Luftzufuhr und den Luftdruck zur Düse.

Die Flüssigkeitsleitung der elektrostatischen Düse wird durch das Sprührohr per Schlauchleitung mit der Pumpe der Rückenspritze verbunden. Ein oder zwei variable Druck- und Flussregulierventil - Hähne steuern den Flüssigkeitsdruck und die Flüssigkeitsmenge.

Die Elektrode der Sprühdüse wird an ihrem speziellen Elektroanschluss mit der Stromversorgung und dem Stromregler per Elektrokabel durch das Sprührohr (verg. Zeichnung A 5) verbunden und mit dem An- und Ausschalt - Kreis eines Schalters verbunden. (verg. Zeichnungen E 1 und E 2)

Zur Überprüfung der Konzentration der elektrolytisch hergestellten oxidativen Radikale kann ein Redoxmeter mit Sonde im Rückenspritzentank oder im Auffangtank der Elektrolyseanlage eingebaut werden.

Mit der ausgerüsteten Spritze kann nun die Behandlung der Pflanzen beginnen.

In den Rückenspritzentank resp. in den Auffangtank wird sauberes Leitungswasser gefüllt.(verg. Zeichnung A 1). Die entsprechenden Salze werden dem Wasser in der richtigen Konzentration für die spezielle Anwendung im aufgerührten Zustand beigegeben.

Die Elektrolyse der salzhaltigen Lösung wird durch den Pumpenschalter in Funktion gesetzt. Salzhaltiges Wasser wird durch die Elektrodenplatten Anode und Kathode gepresst und gleichzeitig mit vorzugsweise ca.30 A und 45 V oder je nach Bedarf mit anderen Intensitäten elektrolysiert, dabei entstehen hoch-oxidative Radikale im Wasser, die zur Desinfektion, respektive zur Zerstörung von Pathogenen auf Pflanzen und Insekten führen.

Diese Salze sind zum Beispiel pro Liter Spritzflüssigkeit: (für Jungpflanzen)

1.5 gr. NaCl (Kochssalz)

0.3 gr. K2S04 (Kaliumsulfat)

0.3 gr. Na3P04 (Natriumphosphat)

0.3 gr Mg2S04 (Magnesiumphosphat)

Diese Salzlösung muss nach der erfolgten Elektrolyse eine Konzentration von min. 35 ppm oder 35mg pro Liter oxidative Radikale als Gesamttotal aufweisen oder ca. 17 ppm oder 17 mg pro Liter freie Radikale enthalten.

Diese Salze sind zum Beispiel pro Liter Spritzflüssigkeit: (für im Wachstum befindliche und ausgewachsene Pflanzen):

2.25 gr. NaCl (Kochssalz)

0.45 gr. K2S04 (Kaliumsulfat)

0.45 gr. Na3P04 (Natriumphosphat)

0.4 gr Mg2S04 (Magnesiumphosphat)

Diese Konzentrationen können je nach Pflanzenart und Wachstumsstadium und Infektionsdruck verschieden sein!

Diese Salzlösung muss nach der erfolgten Elektrolyse eine Konzentration von min. 90 ppm oder 90mg pro Liter oxidative Radikale als Gesamttotal aufweisen oder ca. 45 ppm oder 45 mg pro Liter freie Radikale enthalten.

Die Konzentration der oxidativen Lösung wird entweder mit einem Redoxmeter-Gerät oder chemisch lichtoptisch mit der DPD - Analysemethode analysiert. Je nach Bautyp und Kapazität der Elektrolyseanlage ergeben sich auch Anwender= Tabellen, die zur Bestimmung und der Errechnung der oxidativen Radikalen-Konzentration dienen können, was erfahrungsgemäss einfacher ist. Dabei ist folgende Rechnungsgleichung relevant:

$$C = \frac{\text{ppm des Wassers x Ampères x Elektrolysenzeit}}{\text{Log 5}}$$

Wenn die Konzentration der oxidativen Radikale in der Wasserlösung erreicht ist, kann der Spritvorgang beginnen resp. kann der Tank der Rückenspritze aus dem Auffangtank gefüllt werden. Wenn der Motor der Rückenspritze angesprungen (verg. Zeichnung A 2) ist und die Pumpe und das Gebläse (verg. Zeichnung A 3) auf den korrekten Tourenzahlen laufen, kann die UVC-Lampe (verg. Zeichnung D) angeschaltet werden und die Spritzarbeit kann beginnen.

Der Sprühnebel von maximum 50 Mikron Tröpfchengrösse, der durch die eingeschaltete elektrostatische Düse (verg. Zeichnung A 4) erzeugt wird, muss jetzt so auf die Pflanzen zerstäubt werden, dass die Pflanzen überfall komplett genisst werden können. Erst bei einer vollständigen totalen Benetzung ist der Desinfektionseffekt 100%.

Bei dieser Spritzarbeit ist vor allem zu beachten, dass alle Pflanzenteile auch mit dem UVC-Licht erreicht werden können. Dabei ist zu beachten, dass die UVC-Licht - Expositionszeit der Pflanzen vorzugsweise nicht länger als drei Sekunden beträgt, da die Pflanzen ansonsten Verbrennungen und Nekrosen erleiden könnten.

Nach dem Abtrocknen der Pflanzenblätter ist die Desinfektion abgeschlossen.

Mit dem UVC-Licht werden ebenfalls Keime abgetötet und eine SAR (Systemic Acquired Resistance) ausgelöst, die nach ca. drei Stunden in der Pflanze ihre Wirkungen zeigt und die Pflanze gegen ihre Schädlinge von innen schützen hilft.

Dieser eigene Immunschutz der Pflanzen kann durch regelmässige Spritzbehandlungen allmählich und stetig gesteigert werden.

Spritzungen im Intervall von nieben Tagen haben sich, unter normalen meteorologischen Bedingungen, in zahlreichen Versuchen als ideal erwiesen. Kürzere Spritz-Intervalle oder längere Intervalle sind abhängig vom jeweiligen pathogenen Infektionsdruck, der individuell vom Produzenten oder Anwender in Erfahrung gebracht werden muss und immer von mehreren Parametern abhängt und vor allem auch von der Pflanzenart.

Die Aufwandmenge der Spritzbrühe richtet sich in der Anfangsphase der Kultur je nach Pflanzenart und Pflanzenbestand vorzugsweise von 100 Liter Elektrolyten-Brühe pro Hektar; bei Grosskulturen bis 400 Liter und bei Obst und Baumkulturen bis 2000 L pro Hektare und mehr reichen kann.

Die erfindungsgemässe Pflanzenschutztechnik ist vor allem eine präventive und biodynamische Technik, die vom verantwortungsbewussten und gut ausgebildeten Gartenbesitzer, Gärtner oder landw. Produzenten erfolgreich angewendet werden kann. Das erfindungsgemässe Spritzverfahren ist rückstandsfrei, ohne toxischen Nebenprodukte auf und in Pflanzen und ist auch für den Bio-Anbau äusserst geeignet und zudem sehr Umwelt freundlich und auch für den Anwender ungefährlich.

Die neue Pflanzenschutztechnologie ist, abgesehen von der Erstinvestition der Geräteanschaffung viel günstiger als konventionelles Spritzen mit toxischen Chemieprodukten, die sehr teuer und giftig sind für Mensch, Tier und Umwelt.

Ein Sprühgerät mit der eingebauten Elektrolysentechnik und der UVC-Lampe und der eingebauten Elektrostatischen Sprühtechnologie ist je nach Einsatzintensität sehr rasch amortisiert, da auf den Kauf von teuren Chemikalien verzichtet werden kann.

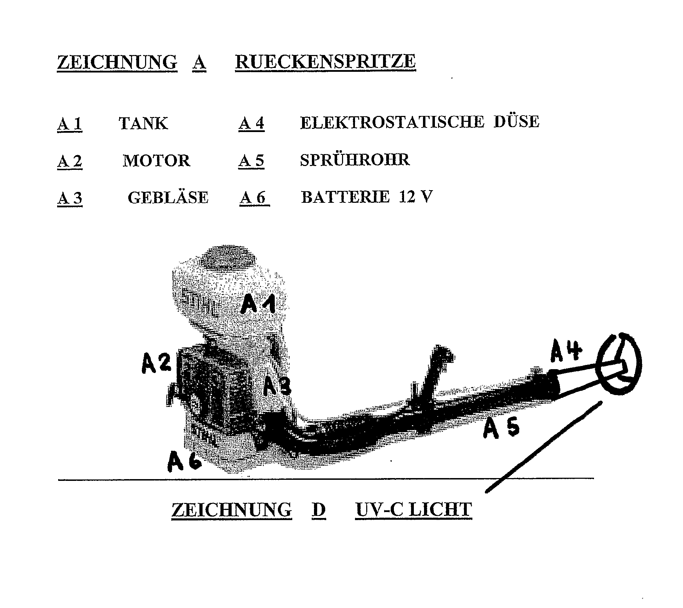

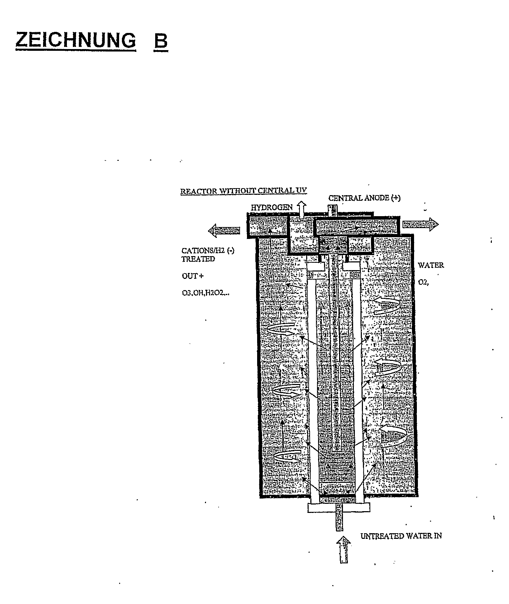

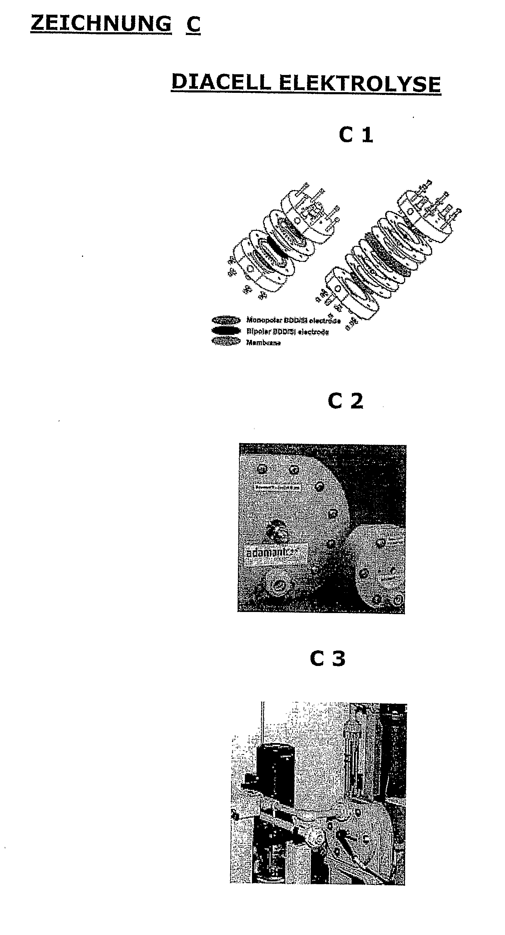

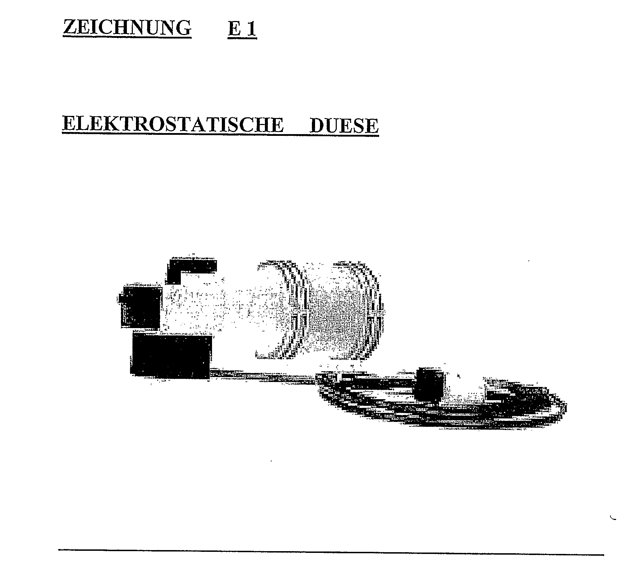

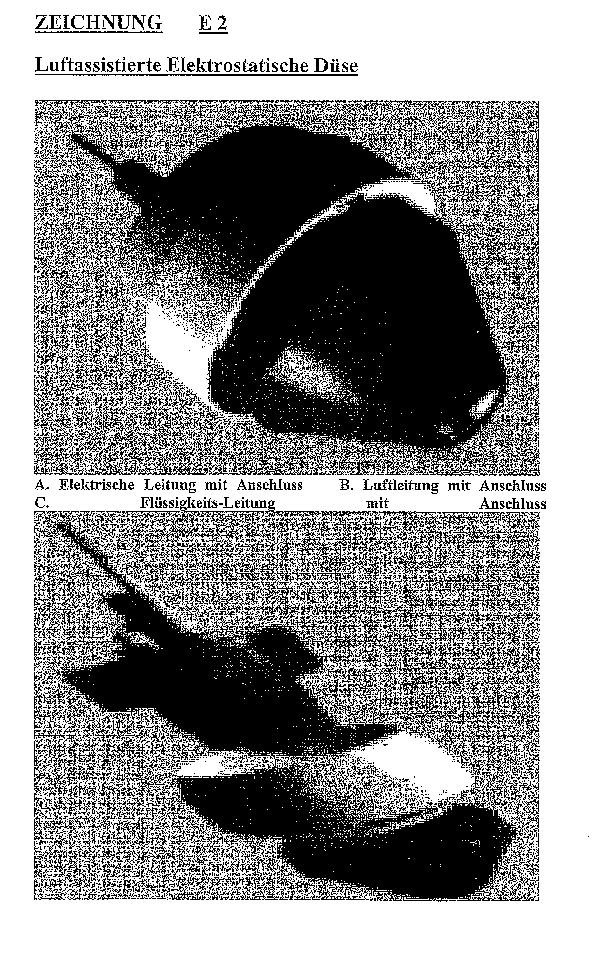

**Patentansprüche**

1.  Spritzverfahren im Pflanzenschutz zur Kontrolle und Elimination von Pilz-, Hefe-, Bakterien-, Virus- und Insektenbefall auf Pflanzen mittels in salzhaltigem Wasser elektrolytisch erzeugter oxidativer Radikale, UVC-Licht-Bestrahlung und mit Hilfe luftassistierter elektrostatischer Spraytechnologie, wobei als Biozid die in salzhaltigem Wasser elektrolytisch erzeugten oxidativen Radikale verwendet werden, die in einer Elektrolysezelle mit diamantbeschichteten Elektroden und/oder in einer Zylinder-Elektrolysezelle mit diaphragma- und platinbeschichteten Elektroden oder kombiniert in beiden produziert werden, wobei die biozide wässrige Lösung von oxidativen Radikalen auf die zu behandelnden Pflanzen mit Hilfe der luftassistierten elektrostatischen Spraytechnologie bei gleichzeitiger UVC-Licht-Direktbestrahlung auf die Pflanzen aufgesprüht wird.

2.  Spritzverfahren im Pflanzenschutz nach Anspruch 1, **dadurch gekennzeichnet, dass** das Verfahren UVC-Licht mit Wellenlängen von 254 nm und 185 nm als zusätzliches Biozid in Form von Direktstrahlung und als Reaktionskatalysator für eine Oxidation von organischer Substanz sowie als Insektenfalle und als SAR (Systemic Acquired Resistance) auslösenden Stressor verwendet.

3.  Spritzverfahren im Pflanzenschutz nach Anspruch 1 oder 2, **dadurch gekennzeichnet, dass** das Verfahren zur vollständigen Benetzung der Pflanzen und Oberflächen eine elektrostatische Spraytechnologie mit luftassistierten Düsen verwendet, welche eine oder mehrere Elektroden enthalten, die durch Induktion eine Ladung von mindestens 600 V oder 3 bis 5 mOhm pro Liter auf die Wassertröpfchen mit einer maximalen Größe von 50 Mikron ermöglichen.

4.  Spritzverfahren im Pflanzenschutz nach einem der Ansprüche 1 bis 3, **dadurch gekennzeichnet, dass** das Verfahren zur Erzeugung der oxidativen Radikale im Wasser folgende Salze in den angegebenen Konzentrationen pro Liter Spritzflüssigkeit verwendet:

    Für Jungpflanzen: 1,5 g NaC1 (Kochsalz), 0,3 g K2S04 (Kaliumsulfat), 0,3 g Na3P04 (Natriumphosphat), 0,5 g Mg2S04 (Magnesiumphosphat), wobei die Salzlösung nach der erfolgten Elektrolyse eine Konzentration von mindestens 35 ppm oder 35 mg/l oxidative Radikale als Gesamttotal aufweist oder ca. 17 ppm bzw. 17 mg/l freie Radikale enthält;
    Für im Wachstum befindliche und ausgewachsene Pflanzen: 2,25 g NaC1 (Kochsalz), 0,45 g K2S04 (Kaliumsulfat), 0,45 g Na3P04 (Natriumphosphat), 0,6 g Mg2S04 (Magnesiumphosphat), wobei die Salzlösung nach der erfolgten Elektrolyse eine Konzentration von mindestens 90 ppm oder 90 mg/l oxidative Radikale als Gesamttotal aufweist oder ca. 45 ppm bzw. 45 mg/l freie Radikale enthält.

5.  Spritzverfahren im Pflanzenschutz nach einem der Ansprüche 1 bis 4, **dadurch gekennzeichnet, dass** die elektrolytisch erzeugten oxidativen Radikale in zwei verschiedenen Elektrolysegeräten erzeugt werden, dass die Pflanzen mittels der elektrolytisch erzeugten oxidativen Radikale und des UVC-Lichts desinfiziert werden, respektive die mikrobielle Last der Pathogene reduziert wird, dass durch das UVC-Licht als Katalysator eine Oxidation der Pathogene erfolgt, dass zusätzlich das UVC-Licht als Insektenfalle mittels hoher Strahlendosis eine tödliche Wirkung entfaltet und dass das UVC-Licht als Stressor eine Genelizitierte systemische Immunreaktion in den Pflanzen auslöst, die zur SAR (Systemic Acquired Resistance) oder zu einer systemisch erlangten Resistenz gegenüber Pathogenen in den Pflanzen führt und dass die luftassistierte elektrostatische Spraytechnik zur vollständigen Benetzung aller Pflanzenteile angewendet wird, damit eine genügende Desinfektion und Vernichtung aller Pathogene durch die oxidativen Radikale auf den Pflanzen erfolgen kann.

6.  Vorrichtung zur Kontrolle und Elimination von Pilz-, Hefe-, Bakterien-, Virus- und Insektenbefall auf Pflanzen mittels in salzhaltigem Wasser elektrolytisch erzeugter oxidativer Radikale, UVC-Licht-Bestrahlung und mit Hilfe luftassistierter elektrostatischer Spraytechnologie, umfassend

    - zumindest eine Elektrolysezelle mit diamantbeschichteten Elektroden und/oder zumindest eine Zylinder-Elektrolysezelle mit diaphragma- und platinbeschichteten Elektroden zur Produktion der oxidativen Radikale,
- zumindest eine elektrostatische Spraydüse zum Aufsprühen der die oxidativen Radikale enthaltenden wässrigen Lösung und
- zumindest eine LTVC-Lampe zur Direktbestrahlung der Pflanzen beim Aufsprühen der die oxidativen Radikale enthaltenden wässrigen Lösung.

**7.** Vorrichtung gemäß Anspruch 6, **dadurch gekennzeichnet, dass** sie aus folgenden Komponenten besteht:

a. einer motorisierten Rückenspritze oder einer Handspritze oder einem anderen Spritzentyp,

b. einer oder mehreren elektrostatischen Spraydüsen mit Niederdruck und großvolumiger Luftunterstützung zur Feintropfen-Erzeugung und mit eingebauter Elektrode zur induktiven Aufladung der Spraytropfen mit einer Tropfengröße von idealerweise nicht mehr als 50 Mikron, die eine Aufladung der Mikrotropfen von ca. 600 V oder 3 bis 5 mOhm (Milli-Ohm) pro Liter erlaubt, inklusive der entsprechenden Elektronik zur Erzeugung des DC-Ladungsstroms der Elektroden und automatischer Handsteuerung zur Aktivierung oder Deaktivierung der Elektroden,

c. einer Spraylanze, einem Sprührohr, einem Spraybalken oder einem Spraygebläse, enthaltend eine oder mehrere elektrostatische Spraydüsen in variabler, den Bedürfnissen entsprechender Anordnung, mit lateralem Abstand von vorzugsweise 25 cm,

d. einem oder mehreren Speicher-Wassertanks für die Aufnahme der elektrolytischen oxidativen Radikale im Wasser in den Volumendimensionen der entsprechenden Spritzentypen, idealerweise von 1 Liter bis zu 4000 Litern oder mehr,

e. einer oder mehreren Umwälzpumpen gemäß der zu erbringenden Literleistung pro Stunde mit einer Mindestdruckleistung von 4 bis 10 Atm inklusive elektronischer Steuerung mit "ON"- und "OFF"-Schalter, inklusive oxidationsfreier Leitungen aus Viton, Teflon, PVC oder einem anderen entsprechend geeigneten, nicht oxidierenden Material,

f. einer oder mehreren Sprühpumpen gemäß der zu erbringenden Literleistung pro Stunde mit einer Mindestdruckleistung von 2 bis 10 Atm zur Ausbringung der erforderlichen Sprühmenge von 50 Liter bis 600 Liter pro Hektar oder mehr, mit "ON"- und "OFF"-Schalter, inklusive oxidationsfreier Leitungen aus Viton, Teflon, PVC oder einem anderen entsprechend geeigneten, nicht oxidierenden Material,

g. zwei oder mehreren Druckmanometern und Druckkontrollventilen mit Rücklauffunktion, eines für die Umwälzpumpe und eines für die Sprühpumpe,

h. einem oder mehreren Luftdruckmesser(n) und/oder Luftvolumenmessgeräte(n),

i. zwei oder mehreren Redox-Messgeräten für die Messung der Konzentration der oxidativen Radikale im Tank und in den Zubringerleitungen zu den elektrostatischen Spraydüsen,

j. einem oder mehreren Diacell-Elektrolysezelle(n) mit je ein bis drei oder mehr Elektrolysekammern, je nach Bedarf mit Volumenflussmesser und Fluss-Sonde sowie entsprechendem Steuergerät mit manueller und/oder automatischer Kathoden- und Anoden-Lastumkehr, eingebautem Amperemeter und Voltmeter sowie Lampenfunktionskontrolle mit automatischer Abschaltung ohne Volumenfluss inklusive Leitungen, Anschlüssen, Kontrollhahn sowie Probeentnahmestelle (220 V oder 340 V),

k. einer oder mehreren Zylinder-Elektrolysezelle(n) mit Plattenelektroden und Diaphragmazellentrennung mit Anode und Kathode mit Umkehrfunktion zur Produktion von saurem und basischem Elektrolysewasser mit anionischen und kathionischen oxidativen Radikalen, mit elektrischer Steuerung, Strompulsor und Sicherung mittels Steuergerät mit manueller und automatischer Kathoden- und Anodenlastumkehr, eingebautem Amperemeter und Voltmeter und Lampenfunktionskontrolle, mit automatischer Abschaltung ohne Volumenfluss, inklusive Leitungen, Anschlüssen, Kontrollhahn sowie Probeentnahmestelle (220 V oder 340 V), inklusive Redox-Messgeräte für die anodische und kathodische Elektrolytenflüssigkeit, inklusive elektronischer Mischbatterie zur Einstellung des gewünschten pH-Wertes des elektrolytischen oxidativen Wassers (EOW),

l. einer oder mehreren LTVC-Lampe(n) von 30 Watt bis 330 Watt oder mehr, als Rund-, Oval- oder Stablampe(n) konzipiert, in wasserdichtem Quarzmantel und wasserdicht verschraubt mit in einem Sicherheits- oder Steuergehäuse integrierten elektrischen Vorschaltgeräten, mit Kontrolllampen und "ON"- bzw. "OFF"-Schaltern sowie Alarm-Funktionssteuerung mit Sicherungsschalter inklusive reflektierendem Strahlungsschutz,

m. einem Luftgenerator, als Gebläse oder Kompressor konzipiert, mit Leitungen und Zapfwellen- oder elektrischem oder motorisiertem Antrieb je nach Leistungsbedarf, inklusive Druck- und Volumenmeter und entsprechenden Leitungen sowie Kühlaggregaten mittel Kühlradiator sowie

n. einer Stromquelle aus der Steckdose, einer Batterie, einer Sonnenenergieversorgungsanlage oder einem Stromgenerator, individuell oder per Zapfwellenantrieb erzeugt, inklusive Steuerungen und Sicherungen.

**Claims**

1. Method of spraying in plant protection for controlling and eliminating fungal, yeast attacks, bacterial and viral infections and insect infestations on plants by means of oxidative radicals produced electrolytically in salt-containing water, UV-C light irradiation and with the aid of air-assisted electrostatic spray technology, wherein the oxidative radicals produced electrolytically in salt-containing water, which are produced in an electrolysis cell with diamond-coated electrodes and/or in a cylinder electrolysis cell with diaphragm and platinum-coated electrodes or combined in both, are used as biocide, wherein the biocidal aqueous solution of oxidative radicals is sprayed on to the plants to be treated with the aid of air-assisted electrostatic spray technology with simultaneous direct UV-C light irradiation.

2. Method of spraying in plant protection in accordance with claim 1, **characterised in that** the method applies UV-C light with wavelengths of 254 nm and 185 nm as additional biocide in the form of direct irradiation and as reaction catalyst for an oxidation of organic matter as well as insect trap and stressor initiating SAR (Systemic Acquired Resistance).

3. Method of spraying in plant protection in accordance with claim 1 or 2, **characterised in that** the method uses an electrostatic spray technology with air-assisted nozzles for complete wetting of the plants and surfaces, which includes one or a plurality of electrodes which, by induction, enable a charge of at least 600 V or 3 to 5 mohm per litre on water droplets with a maximum size of 50 microns.

4. Method of spraying in plant protection in accordance with one of the claims 1 to 3, **characterised in that** the method for production of oxidative radicals uses the following salts in the stated concentration per litre of spraying liquid:

   For young plants: 1.5 g NaCl (common salt), 0.3 g $K_2SO_4$ (potassium sulphate), 0.3 g $Na_3PO_4$ (sodium phosphate), 0.5 g $Mg_2SO_4$ (magnesium phosphate), wherein,
   following electrolysis, the salt solution contains a concentration of at least 35 ppm or 35 mg/l oxidative radicals as overall total or approx. 17 ppm or 17 mg/l free radicals;
   For plants during growth and fully grown plants: 2.25 g NaCl (common salt), 0.45 g $K_2SO_4$ (potassium sulphate), 0.45 g $Na_3PO_4$ (sodium phosphate), 0.6 g $Mg_2SO_4$ (magnesium phosphate) wherein, following electrolysis, the salt solution contains a concentration of at least 90 ppm or 90 mg/l oxidative radicals as overall total or approx. 45 ppm or 45 mg/l free radicals.

5. Method of spraying in plant protection in accordance with one of the claims 1 to 4, **characterised in that** the electrolytically produced oxidative radicals are produced in two different electrolysis units, that the plants are disinfected by means of the electrolytically produced oxidative radicals and the UV-C light, or respectively the microbial load of pathogens is reduced, that oxidation of the pathogens takes place due to the UV-C light as catalyst, that additionally the UV-C light as insect trap develops a lethal action due to a high radiation dose and that the UV-C light as stressor initiates a gene-elicited systemic immune reaction in the plants, leading to the SAR (Systemic Acquired Resistance) or to a systemically acquired resistance to pathogens in the plants and that the air-assisted electrostatic spraying technique is applied for the complete wetting of all parts of the plants, so that adequate disinfection and extermination of all pathogens by the oxidative radicals on the plants can take place.

6. Device for controlling and eliminating fungal, yeast attacks, bacterial and viral infections and insect infestations on plants by means of oxidative radicals produced electrolytically in salt-containing water, UV-C light irradiation and with the aid of air-assisted electrostatic spray technology, comprising

   - at least one electrolysis cell with diamond-coated electrodes and/or at least one cylinder electrolysis cell with diaphragm and platinum-coated electrodes for production of the oxidative radicals,
   - at least one electrostatic spray nozzle for spraying on the aqueous solution containing the oxidative radical-containing aqueous solution and
   - at least one UV-C lamp for direct irradiation of the plants during spraying of the aqueous solution containing the oxidative radicals.

7. Device in accordance with claim 6, **characterised in that** it consists of the following components:

   a. a motorised backpack sprayer or a hand sprayer or another type of sprayer,
   b. one or a plurality of electrostatic spray nozzles with low pressure and large-volume air support for production of fine droplets and with electrode incorporated for inductive charging of the spray droplets having a droplet size ideally not more than 50 microns, allowing the charging of the micro-droplets of approx. 600 V or 3 to 5 mohm (milliohm) per litre, inclusive of the corresponding electronics for generation of the DC charging current of the electrodes and automatic manual control for activation or deactivation of the electrodes,

c. a spray lance, a spray pipe, a spray bar or a spray blower, containing one or a plurality of electrostatic spray nozzles in a variable arrangement, depending on requirements, with lateral spacing preferably of 25 cm,

d. one or a plurality of water storage tanks to hold the electrolytic oxidative radicals in the water in the volumetric dimensions of the corresponding sprayer types, ideally from 1 litre up to 4000 litres or more,

e. one or a plurality of circulation pumps corresponding to the litres per hour output to be supplied with a minimum pressure output of 4 to 10 Atm including electronic control unit with 'ON' and 'OFF' switch, including oxidation-free piping of Viton, Teflon, PVC or another appropriately suitable non-oxidising material,

f. one or a plurality of spray pumps according to litres per hour output to be supplied, with a minimum pressure output of 2 to 10 Atm for output of the required spray volume of 50 litres to 600 litres per hectare or more, with 'ON' and 'OFF' switch, including oxidation-free piping of Viton, Teflon, PVC or another appropriately suitable non-oxidising material,

g. two or a plurality of pressure gauges and pressure control valves with return function, one for the circulating pump and one for the spray pump,

h. one or a plurality of air pressure gauge(s) and/or air flow gauge(s),

i. two or a plurality of redox meters for measuring the concentration of the oxidative radicals in the tank and in the supply lines to the electrostatic spray nozzles,

j. one or a plurality of DiaCell electrolysis cell(s), each with one to three or more electrolysis chambers, each as required with flow meter and flow probe together with corresponding control unit with manual and/or automatic cathode and anode charge reversal, integrated ammeter and voltmeter and lamp function monitoring with automatic switch-off in the absence of flow, including pipelines, connections, control tap and sampling point (220 V or 340 V),

k. one or a plurality of cylinder-electrolysis cell(s) with plate electrodes and diaphragm cell separation with anode and cathode with reversal function for production of acidic and basic electrolysis water containing anionic and cationic oxidative radicals, with electric control system, current pulser and protection by means of control unit with manual and automatic cathode and anode charge reversal, integrated ammeter and voltmeter and lamp function monitor, with automatic disconnection without flow, including pipelines, connections, control tap and sampling point (220 V or 340 V), including redox meter for the anodic and cathodic electrolyte liquid, including electronic mixing tap for adjustment of the desired pH value of the electrolytic oxidative water (EOW),

l. one or a plurality of UV-C lamp(s) of 30 watt to 330 watt or more, designed as round, oval or tubular lamp(s), in watertight quartz sheath and screwed together to be watertight with ballast units integrated in a safety or control housing, with indicator lamps and 'ON'- and 'OFF 'switches together with alarm function control unit with safety cut-off including reflecting radiation protection,

m. an air generator, designed as blower or compressor, with lines and power take-off shafts or electric or motorised drive corresponding to output requirement, including pressure and flow rate gauge and corresponding leads together with cooling units by means of cooling radiators, and

n. a power source from the electrical outlet, a battery, a solar energy supply unit or a power generator, produced individually or via power take-off drive, including control units and safety devices.

## Revendications

1. Procédé d'injection en protection des végétaux pour le contrôle et l'élimination des infestations par des champignons, levures, bactéries, virus et insectes sur des végétaux au moyen de radicaux oxydatifs produits par électrolyse dans de l'eau salée, irradiation à la lumière UVC et à l'aide d'une technologie de pulvérisation électrostatique à assistance pneumatique, dans lequel on utilise comme biocide les radicaux oxydatifs produits par électrolyse dans de l'eau salée, qui sont produits dans une cellule électrolytique avec des électrodes plaquées au diamant et/ou dans une cellule électrolytique à cylindres avec des électrodes plaquées d'un diaphragme ou d'une platine ou en combinaison dans les deux, dans lequel la solution biocide aqueuse de radicaux oxydatifs est pulvérisée sur les végétaux à traiter à l'aide de la technologie de pulvérisation électrostatique à assistance pneumatique tout en irradiant directement les végétaux à la lumière UVC.

2. Procédé d'injection en protection des végétaux selon la revendication 1, **caractérisé en ce que** le procédé utilise de la lumière UVC de longueurs d'onde de 254 nm et 185 nm comme biocide complémentaire sous la forme d'une irradiation directe et comme catalyseur réactif pour une oxydation de substance organique et comme piège à insectes et facteur de stress déclenchant une SAR (Systemic Acquired Resistance : résistance systémique acquise).

EP 2 152 324 B1

3. Procédé d'injection en protection des végétaux selon la revendication 1 ou 2, **caractérisé en ce que** le procédé, pour mouiller entièrement les végétaux et les surfaces, utilise une technologie de pulvérisation électrostatique avec des buses à assistance pneumatique qui contiennent une ou plusieurs électrodes qui permettent par induction une charge d'au moins 600 V ou 3 à 5 mOhm par litre sur les gouttelettes d'eau ayant une taille maximale de 50 microns.

4. Procédé d'injection en protection des végétaux selon une des revendications 1 à 3, **caractérisé en ce que** le procédé de production des radicaux oxydatifs dans l'eau utilise les sels suivants dans les concentrations indiquées par litre de liquide injecté :

pour les jeunes végétaux : 1,5 g de NaCl (sel de cuisine), 0,3 g de K2S04 (sulfate de potassium), 0,3 g de Na3P04 (phosphate de sodium), 0,5 g Mg2S04 (phosphate de magnésium), la solution salée présentant, une fois l'électrolyse faite, une concentration d'au moins 35 ppm ou 35 mg/l de radicaux oxydatifs au total ou contenant environ 17 ppm ou 17 mg/l de radicaux libres ;
pour les plantes en croissance ou qui ont déjà poussé : 2,25 g de NaCl (sel de cuisine), 0,45 g de K2S04 (sulfate de potassium), 0,45 g de Na3P04 (phosphate de sodium), 0,6 g de Mg2S04 (phosphate de magnésium), la solution salée présentant, une fois l'électrolyse faite, une concentration d'au moins 90 ppm ou 90 mg/l de radicaux oxydatifs au total ou contenant environ 45 ppm ou 45 mg/l de radicaux libres.

5. Procédé d'injection en protection des végétaux selon une des revendications 1 à 4, **caractérisé en ce que** les radicaux oxydatifs produits par électrolyse sont produits dans deux électrolyseurs différents, que les plantes sont désinfectées au moyen des radicaux oxydatifs produits par électrolyse et de la lumière UVC ou que la charge microbienne des pathogènes est réduite, qu'une oxydation des pathogènes par la lumière UVC faisant office de catalyseur a lieu, que la lumière UVC faisant office de piège à insectes déploie en outre un effet létal au moyen d'une forte dose de rayons et que la lumière UVC déclenche en tant que facteur de stress dans les végétaux une réaction immunitaire à réplique génique qui entraîne une SAR (Systemic Acquired Resistance : résistance systémique acquise) ou une résistance acquise systémiquement aux pathogènes dans les végétaux et que la pulvérisation électrostatique à assistance pneumatique est utilisée pour mouiller complètement toutes les parties des végétaux pour qu'il puisse y avoir une désinfection et destruction suffisante de tous les pathogènes par les radicaux oxydatifs sur les végétaux.

6. Dispositif de contrôle et d'élimination des infestations par des champignons, levures, bactéries, virus et insectes sur des végétaux au moyen de radicaux oxydatifs produits par électrolyse dans de l'eau salée, irradiation à la lumière UVC et à l'aide d'une technologie de pulvérisation à assistance pneumatique, comprenant

- au moins une cellule électrolytique avec des électrodes plaquées au diamant et/ou au moins une cellule électrolytique à cylindres avec des électrodes plaquées d'un diaphragme ou d'une platine pour la production des radicaux oxydatifs
- au moins une buse de pulvérisation électrostatique pour pulvériser la solution aqueuse contenant les radicaux oxydatifs et
- au moins une lampe UVC pour l'irradiation directe des végétaux lors de la pulvérisation de la solution aqueuse contenant les radicaux oxydatifs.

7. Dispositif selon la revendication 6, **caractérisé en ce qu'**il est constitué des composants suivants :

a. une boille à dos motorisée ou un pulvérisateur manuel ou un autre type de pulvérisateur,
b. une ou plusieurs buses de pulvérisation électrostatiques à basse pression et assistance pneumatique de gros volume pour la création de gouttelettes et avec électrode intégrée pour le chargement inductif des gouttes pulvérisées ayant une taille de gouttes ne dépassant pas 50 microns dans l'idéal, qui permet une charge des microgouttes d'environ 600 V ou 3 à 5 mOhm (milli-Ohms) par litre, y compris le système électronique correspondant pour générer le courant de charge DC des électrodes et la commande manuelle automatique pour activer ou désactiver les électrodes,
c. une lance de pulvérisation, un tuyau de pulvérisation, une poutre de pulvérisation ou une soufflerie de pulvérisation, contenant une ou plusieurs buses de pulvérisation électrostatiques en agencement variable répondant aux besoins, avec un espacement latéral de préférence de 25 cm,
d. une ou plusieurs citernes à eau destinées à recevoir les radicaux oxydatifs électrolytiques dans l'eau dans les dimensions volumiques des types de pulvérisateurs correspondants, dans l'idéal de 1 litre à 4000 litres ou plus,
e. une ou plusieurs pompes de circulation suivant la capacité en litres à fournir par heure avec une capacité de pression minimale de 4 à 10 Atm, y compris commande électronique avec interrupteurs "ON" et "OFF", y compris des tuyaux antioxydants en Viton, Teflon, PVC ou un autre matériau approprié en conséquence et non oxydant,

f. une ou plusieurs pompes de pulvérisation suivant la capacité en litres à fournir par heure avec une capacité de pression minimale de 2 à 10 Atm pour diffuser la quantité de pulvérisation nécessaire de 50 litres à 600 litres par hectare ou plus, avec interrupteur "ON" et "OFF", y compris des tuyaux antioxydants en Viton, Teflon, PVC ou un autre matériau approprié en conséquence et non oxydant,

g. deux ou plusieurs manomètres de pression et soupapes de contrôle de pression à fonction reflux, un pour la pompe de circulation et un pour la pompe de pulvérisation,

h. un ou plusieurs mesureurs de pression atmosphérique et/ou appareils de mesure du volume d'air,

i. deux ou plusieurs appareils de mesure Redox pour mesurer la concentration des radicaux oxydatifs dans la citerne et dans les conduites d'alimentation des buses de pulvérisation électrostatiques,

j. une ou plusieurs cellules électrolytiques Diacell comportant chacune une à trois ou plus de chambres électrolytiques, suivant les besoins avec mesureur de débit volumique et sonde de flux et appareil de commande correspondant avec inversion manuelle et/ou automatique de charge cathodique et anodique, ampèremètre intégré et voltmètre, de même que contrôleur de fonctionnement des lampes avec coupure automatique sans débit volumique, y compris les conduites, raccords, le robinet de contrôle et un point de prélèvement d'échantillons (220 V ou 340 V),

k. une ou plusieurs cellules électrolytiques à cylindre avec électrodes plates et séparation de cellules à diaphragme avec anode et cathode et fonction inversion pour la production d'eau électrolytique acide et basique avec des radicaux anioniques et cationiques, avec commande électrique, impulseur électrique et sécurisation par appareil de commande avec inversion de charge cathodique et anodique manuelle et automatique, ampèremètre intégré et voltmètre, de même que contrôleur de fonctionnement des lampes avec coupure automatique sans débit volumique, y compris les conduites, raccords, le robinet de contrôle et un point de prélèvement d'échantillons (220 V ou 340 V), y compris appareils de mesure Redox pour le liquide électrolytique anodique et cathodique, y compris mitigeur électronique pour le réglage de la valeur de pH souhaitée de l'eau électrolytique oxydative (EEO),

l. une ou plusieurs lampes UVC de 30 watts à 330 watts ou plus, conçues sous forme de lampes, rondes, ovales ou en barres, vissées dans une chemise en quartz étanche à l'eau et vissées avec étanchéité à l'eau sur des ballasts électriques intégrés dans un boîtier de sécurité ou de commande, avec des voyants de contrôle et des interrupteurs "ON" ou "OFF" et une commande de fonctionnement d'alarme avec disjoncteur, y compris système de protection réfléchissant anti-rayonnement,

m. un générateur d'air conçu sous forme d'une soufflerie ou d'un compresseur, avec des conduites et une commande à arbre de prise de force ou électrique ou motorisée suivant la puissance requise, y compris manomètre et débitmètre et des conduites correspondantes, de même que des sous-ensembles de refroidissement par radiateur réfrigérant et

n. une source de courant sur prise électrique, une batterie, une installation d'alimentation en énergie solaire ou un générateur de courant produit individuellement ou par commande à arbre de prise de force, y compris les commandes et fusibles.

## ZEICHNUNG A    RUECKENSPRITZE

A 1    TANK          A 4    ELEKTROSTATISCHE DÜSE

A 2    MOTOR         A 5    SPRÜHROHR

A 3    GEBLÄSE       A 6    BATTERIE 12 V

## ZEICHNUNG D    UV-C LICHT

# ZEICHNUNG  B

REACTOR WITHOUT CENTRAL UV

HYDROGEN

CENTRAL ANODE (+)

CATIONS/H2 (-)
TREATED

OUT +

O3,OH,H2O2...

WATER

O2,

UNTREATED WATER IN

# ZEICHNUNG C

## DIACELL ELEKTROLYSE

### C 1

Monopoler BDD/Si electrode
Bipoler BDD/Si electrode
Membrane

### C 2

### C 3

## ZEICHNUNG  E 1

## ELEKTROSTATISCHE  DUESE

## ZEICHNUNG    E 2

## Luftassistierte Elektrostatische Düse

A. Elektrische Leitung mit Anschluss    B. Luftleitung mit Anschluss
C.      Flüssigkeits-Leitung        mit       Anschluss

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004089075 A2